# EUROPÄISCHE PATENTSCHRIFT

(11) **EP 0 567 793 B1**
(45) Veröffentlichungstag und Bekanntmachung des Hinweises auf die Patenterteilung: **23.10.1996**
(21) Anmeldenummer: 93105337.5
(22) Anmeldetag: 31.03.1993
(51) Int. Cl.: C09B 62/51, C09B 62/085

(54) **Wasserlösliche Azoverbindungen, Verfahren zu ihrer Herstellung und ihre Verwendung als Farbstoffe**
Water-soluble azocompounds, process for their manufacture and their use as dyes
Composés azo, solubles dans l'eau, procédé pour leur préparation et leur utilisation comme colorants

(30) Priorität: 01.04.1992 DE 4210732
(43) Veröffentlichungstag der Anmeldung: 03.11.1993
(73) Patentinhaber: HOECHST AKTIENGESELLSCHAFT, 65926 Frankfurt am Main (DE)
(72) Erfinder: Dannheim, Jörg, Dr., W-6000 Frankfurt/M (DE)

(56) Entgegenhaltungen:
- EP-A- 0 122 881
- EP-A- 0 356 860

## Beschreibung

Die Erfindung liegt auf dem technischen Gebiet der wasserlöslichen Azofarbstoffe, die faserreaktive Eigenschaften besitzen.

Mit der vorliegenden Erfindung wurden neue wasserlösliche Azoverbindungen gefunden, die der allgemeinen Formel (1) 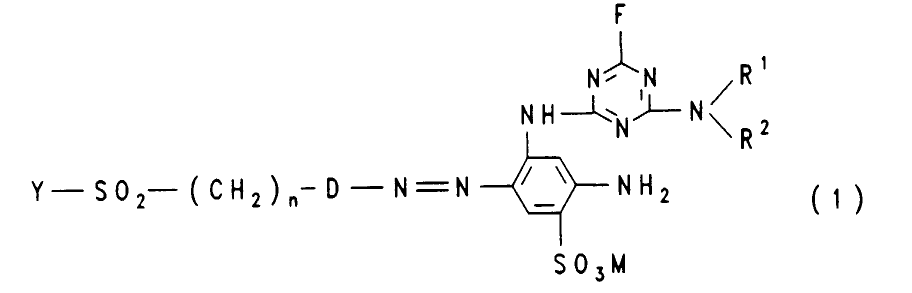 entsprechen, worin bedeuten:
- Y: ist Vinyl oder ist Ethyl, das in ß-Stellung einen Substituenten enthält, der durch ein Alkali unter Bildung der Vinylgruppe eliminierbar ist, oder ist β-Hydroxyethyl;
- n: ist die Zahl Null, 1 oder 2;
- D: ist Phenylen, das durch 1 oder 2 Substituenten aus der Gruppe Alkyl von 1 bis 4 C-Atomen, Alkoxy von 1 bis 4 C-Atomen, Sulfo, Chlor, Brom, Fluor, Carboxy, Nitro, Hydroxy, Alkanoylamino von 2 bis 5 C-Atomen, Benzoylamino und durch Substituenten aus der Gruppe Methyl, Ethyl, Methoxy, Ethoxy, Chlor und Sulfo substituiertes Benzoylamino oder durch 1 oder 2 dieser Substituenten und eine Gruppe der allgemeinen Formel Y-SO₂-(CH₂)ₘ-, in welcher Y eine der oben genannten Bedeutungen hat und m die Zahl 1 oder 2 ist, oder durch eine Gruppe der Formel Y-SO₂-(CH₂)ₘ- substituiert sein kann, oder
- D: ist Naphthylen, das durch 1 oder 2 Substituenten aus der Gruppe Alkyl von 1 bis 4 C-Atomen, Alkoxy von 1 bis 4 C-Atomen, Chlor, Brom, Fluor, Carboxy, Sulfo, Nitro, Hydroxy, Alkanoylamino von 2 bis 5 C-Atomen, Benzoylamino und durch Substituenten aus der Gruppe Alkyl von 1 bis 4 C-Atomen, Alkoxy von 1 bis 4 C-Atomen, Chlor und Sulfo substituiertes Benzoylamino, bevorzugt durch Sulfo, substituiert sein kann, oder
- D: ist Benzthiazol-2-yl, das im carbocyclischen Ring die angegebene Gruppe der Formel Y-SO₂-(CH₂)ₙ- gebunden enthält und in diesem Benzolkern durch einen weiteren Substituenten aus der Gruppe Alkyl von 1 bis 4 C-Atomen, Alkoxy von 1 bis 4 C-Atomen, Sulfo, Nitro, Chlor, Brom, Hydroxy, Alkanoylamino von 2 bis 5 C-Atomen, Benzoylamino und durch Substituenten aus der Gruppe Alkyl von 1 bis 4 C-Atomen, Alkoxy von 1 bis 4 C-Atomen, Chlor und Sulfo, bevorzugt Sulfo, substituiertes Benzoylamino substituiert sein kann;
- M: ist Wasserstoff oder das Moläquivalent eines Metalls, vorzugsweise eines Alkalimetalls oder Erdalkalimetalls, wie des Calciums, und ist insbesondere bevorzugt Natrium, Kalium oder Lithium;
- R¹: ist Wasserstoff oder Alkyl von 1 bis 4 C-Atomen, wie Methyl oder Ethyl, oder ist Phenyl oder ist Alkyl von 1 bis 4 C-Atomen, bevorzugt von 2 bis 4 C-Atomen, das durch 1 oder 2 nicht-ionogene und/oder anionische Substituenten substituiert ist, wie beispielsweise durch Substituenten aus der Gruppe Hydroxy, Alkanoyloxy von 2 bis 5 C-Atomen, Sulfato, Phosphato, Phosphono, Sulfo, Carboxy, die Phosphonsäuregruppe, Alkoxy von 1 bis 4 C-Atomen, Phenyl, Sulfophenyl und Cyano, bevorzugt durch Substituenten aus der Gruppe Sulfo, Carboxy, die Phosphonsäuregruppe, Suffato und Phosphato;
- R²: ist Alkyl von 1 bis 4 C-Atomen, wie Methyl oder Ethyl, oder ist Phenyl oder ist Alkyl von 1 bis 4 C-Atomen, bevorzugt von 2 bis 4 C-Atomen, das durch 1 oder 2 nicht-ionogene und/oder anionische Substituenten substituiert ist, wie beispielsweise durch Substituenten aus der Gruppe Hydroxy, Alkanoyloxy von 2 bis 5 C-Atomen, Sulfato, Phosphato, Phosphono, Sulfo, Carboxy, die Phosphonsäuregruppe, Alkoxy von 1 bis 4 C-Atomen, Phenyl, Sulfophenyl und Cyano, bevorzugt durch Substituenten aus der Gruppe Sulfo, Carboxy, die Phosphonsäuregruppe, Sulfato und Phosphato, oder ist Phenyl, das durch 1,2 oder 3 Substituenten aus der Gruppe Methyl, Ethyl, Methoxy, Ethoxy, Chlor, Cyano, Nitro, Sulfo, Carboxy und Carbamoyl, bevorzugt durch 1 oder 2 Substituenten aus der Gruppe Sulfo und Carboxy, substituiert ist, oder ist Naphthyl, das durch 1,2 oder 3 Sulfogruppen oder durch 1 oder 2 Carboxygruppen oder durch 1 oder 2 Sulfogruppen und eine Carboxygruppe substituiert sein kann, bevorzugt substituiert ist.

Der Formelrest D ist bevorzugt Phenylen, das unsubstituiert oder durch 1 oder 2 Substituenten aus der Gruppe Methyl, Ethyl, Methoxy, Ethoxy, Chlor und Brom, bevorzugt durch 1 oder 2 Substituenten aus der Gruppe Methyl, Methoxy und Ethoxy, substituiert ist, oder ist bevorzugt Naphthylen, wie insbesondere Naphth-2,6-ylen und Naphth-2,8-ylen mit der Bindung zur Azogruppe in 2-Stellung, das unsubstituiert oder durch eine oder zwei, bevorzugt eine, Sulfogruppe substituiert ist.

Bevorzugt ist in Formel (1) R¹ Wasserstoff oder Alkyl von 1 bis 4 C-Atomen, wie Methyl oder Ethyl, und insbesondere bevorzugt Wasserstoff, und bevorzugt ist in Formel (1) R² Alkyl von 2 bis 4 C-Atomen, das durch Carboxy, Sulfo oder durch die Phosphonsäuregruppe substituiert ist, oder ist der Phenylrest, der durch 1 oder 2 Substituenten aus der Gruppe Carboxy, Sulfo und der Phosphonsäuregruppe substituiert ist und durch einen oder zwei weitere Substituenten aus der Gruppe Methyl, Ethyl, Methoxy und Ethoxy substituiert sein kann; insbesondere bevorzugt ist R² durch 1 oder 2 Sulfo substituiertes Phenyl.

Alkyl von 1 bis 4 C-Atomen ist bevorzugt Ethyl und insbesondere Methyl, und Alkoxy von 1 bis 4 C-Atomen ist bevorzugt Ethoxy und insbesondere Methoxy; Alkanoylamino von 2 bis 5 C-Atomen ist bevorzugt Propionylamino und Acetylamino.

Substituenten, die in β-Stellung des Ethylrestes von Y stehen und durch ein Alkali eliminierbar sind, sind beispielsweise Halogenatome, wie Brom, Fluor und insbesondere Chlor, Estergruppen organischer Carbonsäuren oder Sulfonsäuren, beispielsweise Alkanoyloxy von 2 bis 5 C-Atomen, wie Propionyloxy und insbesondere Acetyloxy, oder Alkylsulfonyloxy von 1 bis 4 C-Atomen, wie Ethylsulfonyloxy, oder Acyloxyreste aromatischer Carbon- oder Sulfonsäuren, wie Benzoyloxy, Sulfobenzoyloxy, Phenylsulfonyloxy oder Toluol-sulfonyloxy, desweiteren beispielsweise die Monoestergruppen der Phosphorsäure und insbesondere der Schwefelsäure und der Thioschwefelsäure, d.h. Phosphato und insbesondere Sulfato und Thiosulfato, oder dieser β-ständige Substituent ist beispielsweise Alkylsulfonylamino mit einem Alkylrest von 1 bis 4 C-Atomen oder ein Arylsulfonylaminorest, wie beispielsweise der Phenylsulfonylaminorest, oder ist Phenoxy oder Dialkylamino mit Alkylgruppen von jeweils 1 bis 4 C-Atomen, wie Dimethylamino und Diethylamino. Bevorzugt ist Y Vinyl und insbesondere β-Sulfatoethyl.

Sulfogruppen sind Gruppen entsprechend der allgemeinen Formel -SO₃M , Carboxygruppen sind Gruppen entsprechend der allgemeinen Formel -COOM, Sulfatogruppen sind Gruppen entsprechend der allgemeinen Formel -OSO₃M , Thiosulfatogruppen sind Gruppen entsprechend der allgemeinen Formel -S-SO₃M , Phosphatogruppen sind Gruppen entsprechend der allgemeinen Formel -OPO₃M₂ und Phosphonogruppen sind Gruppen entsprechend der allgemeinen Formel -OPO₂M₂, in welchen M eine der obengenannten Bedeutungen besitzt.

Die erfindungsgemäßen Azoverbindungen können gemäß der Definition von M sowohl in Form der freien Säure als auch in Form ihrer Salze vorliegen. Bevorzugt sind sie in Form der Salze, insbesondere der Alkalimetallsalze, wie Natrium-, Kalium- und Lithiumsalze. Die erfindungsgemäßen Azoverbindungen finden bevorzugt in Form der Salze, bevorzugt Alkalimetallsalze, Verwendung zum Färben und Bedrucken von hydroxy- oder carbonamidgruppenhaltigen Materialien, insbesondere Fasermaterialien.

Von den erfindungsgemäßen Azoverbindungen können weiterhin diejenigen hervorgehoben werden, die der allgemeinen Formel (1 a) 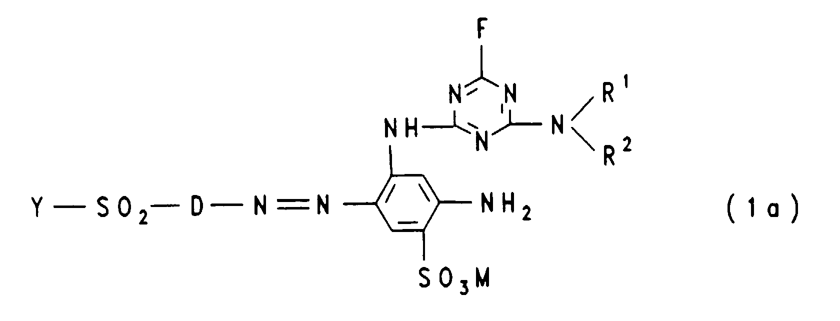 entsprechen, in welcher R¹, R², M und Y eine der obengenannten, insbesondere bevorzugten, Bedeutungen haben, D Phenylen oder durch 1 oder 2 Substituenten aus der Gruppe Methyl, Ethyl, Methoxy, Ethoxy, Chlor und Brom, bevorzugt Methyl, Methoxy und Ethoxy, substituiertes Phenylen oder Naphthylen, bevorzugt 2,6- oder 2,8-Naphthylen, das durch eine Sulfogruppe substituiert sein kann. Bevorzugt ist in Formel (1 a) R¹ Wasserstoff und R² Monosulfo- oder Disulfo-phenyl. Bevorzugt ist der Rest D in den Verbindungen der allgemeinen Formel (1) mit n gleich Null und in den Verbindungen der allgemeinen Formel (1 a) der p-Phenylenrest.

Die vorliegende Erfindung betrifft weiterhin ein Verfahren zur Herstellung der erfindungsgemäßen Azoverbindungen der allgemeinen Formel (1), das dadurch gekennzeichnet ist, daß man die Diazoniumverbindung einer Aminoverbindung der allgemeinen Formel (2)

Y - SO₂ - (CH₂)ₙ - D - NH₂ (2)

in welcher D, Y und n eine der obengenannten Bedeutungen besitzt, mit einer Verbindung der allgemeinen Formel (3) 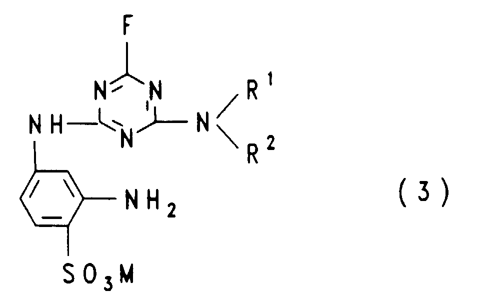 in welcher R¹, R² und M eine der obengenannten Bedeutungen besitzt, kuppelt und gewünschtenfalls im Falle, daß Y β-Hydroxyethyl ist, die Gruppe Y-SO₂- in üblicher Weise mit Hilfe eines Sulfatierungsmittels in die Azoverbindung der allgemeinen Formel (1) mit Y gleich β-Sulfatoethyl oder mittels eines Phosphatierungsmittels in die Azoverbindung der allgemeinen Formel (1) mit Y gleich β-Phosphatoethyl überführt. Sulfatierungsmittel sind beispielsweise 90 bis 100 %ige Schwefelsäure, Chlorsulfonsäure, Amidosulfonsäure oder Schwefeltrioxid-abgebende Verbindungen, wie Schwefeltrioxid-enthaltende Schwefelsäure (Oleum). Phosphatierungsmittel sind beispielsweise konzentrierte Phosphorsäure, Pyrophosphorsäure, Metaphosphorsäure oder Polyphosphorsäure, desweiteren Polyphosphorsäurealkylester, Phosphoroxychlorid oder Gemische aus Phosphorsäure und Phosphor(V)-oxid.

Die erfindungsgemäße Kupplungsreaktion wird analog bekannten Verfahrensweisen der Kupplung von Diazoniumsalzen mit Anilinverbindungen durchgeführt, so in neutralem bis stark saurem, vorzugsweise wäßrigem Milieu, bevorzugt bei einem pH-Wert zwischen 1 und 7, vorzugsweise zwischen 3,5 und 6,5, und bei einer Temperatur zwischen -5°C und + 25°C, vorzugsweise zwischen 5 und 20°C. Puffersubstanzen, wie Natriumacetat und Natriumdihydrogenphosphat, und die Kupplungsgeschwindigkeit verbessernde Verbindungen, wie beispielsweise Dimethylformamid und Pyridin, können dem Reaktionsansatz zur Herstellung der erfindungsgemäßen Azoverbindungen zugesetzt werden.

Die Überführung der β-Hydroxyethylsulfonylgruppe in eine β-Sulfato- oder β-Phosphatoethylsuffonyl-Gruppe erfolgt bei einer Temperatur zwischen 0°C und +80°C, abhängig von der Reaktivität des Sulfatierungs- und Phosphatierungsmittels. In der Regel erfolgt die Sulfatierung mittels 96 bis 100 %iger Schwefelsäure oder bis zu 20 % Schwefeltrioxid enthaltender Schwefelsäure bei einer Temperatur zwischen 0 und 25°C.

Aminoverbindungen entsprechend der allgemeinen Formel (2), die als Diazokomponenten zur Herstellung der erfindungsgemäßen Azoverbindungen dienen, sind beispielsweise:
4-β-Sulfatoethylsulfonyl-anilin, 4-β-Chlorethylsulfonyl-anilin, 4-β-Phosphatoethylsulfonyl-anilin, 4-Vinylsulfonyl-anilin, 4-β-Thiosulfatoethylsulfonyl-anilin, 2-Brom-4-β-sulfatoethylsulfonyl-anilin, 2-Chlor-4-β-sulfatoethylsufonyl-anilin, 2-Chlor-5-β-chlorethylsulfonyl-anilin, 3-β-Sulfatoethylsulfonyl-anilin, 2-Brom-5-β-sulfatoethylsulfonyl-anilin, 2,6-Dichlor-4-β-sulfatoethylsulfonyl-anilin, 2,6-Dibrom-4-β-sulfatoethylsulfonyl-anilin, 2,5-Dichlor-4-β-sulfatoethylsulfonyl-anilin, 2-Methyl-5-β-suffatoethylsulfonyl-anilin, 2-Methoxy-5-β-sulfatoethylsulfonyl-anilin, 2-Methoxy-4-β-sulfatoethylsulfonyl-anilin, 2-Methyl-6-chlor-4-β-sulfatoethylsulfonyl-anilin, 2,6-Dimethyl-4-β-sulfatoethylsulfonyl-anilin, 2,6-Dimethyl-3-β-sulfatoethylsulfonyl-anilin, 2,5-Dimethoxy-4-β-sulfatoethylsulfonyl-anilin, 2-Methoxy-5-methyl-4-β-sulfatoethylsulfonyl-anilin, 2-Nitro-4-β-sulfatoethylsulfonyl-anilin, 4-Nitro-2-β-suffatoethylsulfonyl-anilin, 6-β-Sulfatoethylsulfonyl-2-naphthylamin,1-Sulfo-6-β-suffatoethylsulfonyl-2-naphthylamin, 8-β-Sulfatoethylsulfonyl-2-naphthylamin, 6-Sulfo-8-β-sulfatoethylsulfonyl-2-naphthylamin, 6-Vinylsulfonyl-2-aminobenzthiazol, 6-β-Sulfatoethylsulfonyl-2-aminobenzthiazol, 4-ω-(β-Sulfatoethylsulfonyl)-tolylamin, 6-Methoxy-3-ω-(β-sulfatoethylsulfonyl)-tolylamin, 4-Methoxy-3-ω-(β-sulfatoethylsuffonyl)-tolylamin, 4-Methyl-3,5-bis-(β-sulfatoethylsulfonylmethyl)-anilin, 4-β-(β'-Sulfatoethylsulfonyl)-ethyl-anilin, 3-β-(β'-Sulfatoethylsulfonyl)-ethyl-anilin und 5-(β-Sulfatoethylsulfonylmethyl)-1-naphthylamin sowie die β-Hydroryethylsulfonyl-, β-Chlor-, β-Acetoxy- und β-Phosphatoethylsulfonyl- und Vinylsulfonyl-Abkömmlinge dieser Verbindungen, soweit sie bereits noch nicht genannt wurden.

Die Verbindungen der Formel (2) sind bekannt und in der Literatur vielfach beschrieben. Ebenso sind die als Ausgangsverbindungen zur Herstellung der Kupplungskomponente der allgemeinen Formel (3) dienenden Verbindungen (1,3-Phenylen-diamin-6-sulfonsäure, Cyanurfluorid und Amine der allgemeinen Formel HNR¹ R² mit R¹ und R² der obengenannten Bedeutungen) bekannt.

Die Ausgangsverbindungen der allgemeinen Formel (3) lassen sich analog bekannten Verfahrensweisen der Umsetzung von Cyanurfluorid (2,4,6-Trifluor-1,3,5-triazin) mit Aminoverbindungen herstellen. Die Umsetzung kann hierbei in wäßrigem Medium oder in einem wäßrig-organischen Medium durchgeführt werden, wobei der organische Anteil ein mit Wasser mischbares organisches Lösemittel ist, das gegenüber Trifluortriazin inert ist, wie beispielsweise Aceton, Dioxan und Dimethylformamid. So kann man die Verbindung der Formel (3) durch Umsetzung von Trifluortriazin mit einem Amin der allgemeinen Formel HNR¹R² obengenannter Bedeutung bei einer Temperatur zwischen -5°C und +5°C und einem pH-Wert zwischen 2 und 7 und nachfolgender Umsetzung der erhaltenen Difluor-amino-triazin-Verbindung mit der 1,3-Diaminobenzol-6-sulfonsäure bei einer Temperatur zwischen 0 und 10°C und einem pH-Wert zwischen 2 und 7 herstellen. Geht man zuerst von der 1,3-Diaminobenzol-6-sulfonsäure aus und setzt diese mit Cyanurfluorid um, so wählt man eine Reaktionstemperatur zwischen -5°C und + 10°C und einen pH-Wert zwischen 2 und 7; die nachfolgende Umsetzung der Difluortriazinverbindung mit dem Amin HNR¹ R² erfolgt sodann bei einer Temperatur zwischen 0 und 10°C und einem pH-Wert zwischen 2 und 7. Zur Einstellung des pH-Wertes und zur Abpufferung der freiwerdenden Säure verwendet man die üblichen säurebindenden Mittel, wie Alkali- und Erdalkalicarbonate, Natriumhydroxid, Alkali- und Erdalkalihydrogencarbonate, Natriumfluorid und Alkaliacetate, wobei die Alkali- und Erdalkalimetalle vorzugsweise Natrium, Kalium und Calcium sind.

Ausgangsverbindungen der allgemeinen Formel (3) sind beispielsweise
3-[2'-Fluor-4'-(4''-sulfophenyl)-amino-s-triazin-6'-yl]-amino-6-sulfo-anilin,
3-[2'-Fluor-4'-(3''-sulfophenyl)-amino-s-triazin-6'-yl]-amino-6-sulfo-anilin,
3-[2'-Fluor-4'-(2''-sulfophenyl)-amino-s-triazin-6'-yl]-amino-6-sulfo-anlin,
3-[2'-Fluor-4'-(β-sulfoethyl)-amino-s-triazin-6'-yl]-amino-6-sulfo-anilin,
3-[2'-Fluor-4'-(3''-carboxyphenyl)-amino-s-triazin-6'-yl]-amino-6-sulfo-anilin,
3-[2'-Fluor-4'-(4''-carboxyphenyl)-amino-s-triazin-6'-yl]-amino-6-sulfo-anilin,
3-[2'-Fluor-4'(2''-carboxyphenyl)-amino-s-triazin-6'-yl]-amino-6-sulfo-anilin,
3-[2'-Fluor-4'-(1''-sulfo-naphth-2''-yl)-amino-s-triazin-6'-yl]amino-6-sulfo-anilin,
3-[2'-Fluor-4'-(4''-carboxy-2''-methyl-phenyl)-amino-s-triazin-6'-yl]-amino-6-sulfo-anilin,
3-[2'-Fluor-4'-(5''-sulfo-2''-chlor-phenyl)-amino-s-triazin-6'-yl]-amino-6-sulfo-anilin und
3-[2'-Fluor-4'-(3''-sulfo-4''-chlor-phenyl)-amino-s-triazin-6'-yl]-amino-6-sulfo-anilin.

Die Abscheidung und Isolierung der erfindungsgemäßen Verbindungen der allgemeinen Formel (1) - im nachfolgenden als Verbindungen (1) bezeichnet - aus den wäßrigen Syntheselösungen kann nach allgemein bekannten Methoden für wasserlösliche Verbindungen erfolgen, so beispielsweise, vorteilhaft nach Einstellen der Syntheselösung auf einen pH-Wert von 3,5 bis 7,0, durch Ausfällen aus dem Reaktionsmedium mittels eines Elektrolyten, wie Natriumchlorid oder Kaliumchlorid, oder durch Eindampfen oder Sprühtrocknung der Syntheselösung. Falls die letzte Art der Isolierung gewählt wird, empfiehlt es sich vielfach, vor dem Eindampfen eventuell in den Lösungen vorhandene Sulfatmengen durch Fällung als Calciumsulfat und Abtrennung durch Filtration zu entfernen.

Die Verbindungen (1) haben faserreaktive Eigenschaften und besitzen sehr wertvolle Farbstoffeigenschaften. Sie können deshalb zum Färben (einschließlich Bedrucken) von hydroxygruppenhaltigen und/oder carbonamidgruppenhaltigen Materialien verwendet werden. Hierzu können die Verbindungen (1) auch in Form der nach der Synthese erhaltenen Lösungen, gegebenenfalls nach Zusatz einer Puffersubstanz und gegebenenfalls nach Konzentrierung, direkt als Flüssigpräparation der färberischen Verwendung zugeführt werden.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, wie beispielsweise in Form von Strängen und Wickelkörpern, und Geweben. Hierbei kann man analog bekannten Verfahrensweisen vorgehen.

Hydroxygruppenhaltige Materialien sind solche natürlichen oder synthetischen Ursprungs, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, beispielsweise Wolle und andere Tierhaare, Seide, Leder, Polyamid-6,6, Polyamid-6, Polyamid-1 1 und Polyamid-4.

Die Verbindungen (1) lassen sich, gemäß der erfindungsgemäßen Anwendung, auf den genannten Substraten, insbesondere auf den genannten Fasermaterialien, nach den für wasserlösliche, faserreaktive Farbstoffe bekannten Anwendungstechniken applizieren und fixieren, so beispielsweise, indem man die Verbindungen (1) in gelöster Form auf das Substrat aufbring oder sie darin einbringt und sie auf oder in diesem durch Hitzeeinwirkung oder durch Einwirkung eines alkalisch wirkenden Mittels oder durch beide Maßnahmen fixiert. Solche Färbe- und Fixierweisen sind in der Literatur zahlreich beschrieben (bspw. in der europäischen Patentanmeldungs-Veröffentlichung Nr. 0 181 585 A2).

Beispielsweise erhält man mit den Verbindungen (1) auf Cellulosefasern nach dem Ausziehverfahren unter Verwendung verschiedenster Alkalizusätze aus langer Flotte Färbungen mit sehr guter Farbausbeute. Mit ebenfalls ausgezeichneten Farbausbeuten färben sie Cellulosefasern nach den bekannten Klotzverfahren, wobei die Verbindung (1) mittels Alkali durch Verweilen bei Raumtemperatur, durch Dämpfen oder mit Trockenhitze fixiert werden kann. Bei den Druckverfahren können ebenfalls die üblichen einphasigen Verfahren in Anwesenheit eines säurebindenden Mittels oder alkalispendenden Mittels, wie beispielsweise Natriumbicarbonat, Natriumcarbonat oder Natriumtrichloracetat, in der Druckpaste mit anschließendem Fixieren durch Dämpfen, beispielsweise bei 101 bis 103°C, oder die zweiphasigen Verfahren unter Verwendung neutraler oder schwach saurer Druckpasten, nach deren Druck das Fasermaterial entweder durch ein heißes elektrolythaltiges alkalisches Bad geführt oder aber mit einer alkalischen elektrolythaltigen Klotzflotte überklotzt und die Verbindung (1) danach durch Dämpfen oder Trockenhitze fixiert wird, verwendet werden. Man erhält mit diesen Verfahren farbstarke Drucke mit gutem Stand der Konturen und einem klaren Weißfond. Die Qualität der Drucke wird von wechselnden Fixierbedingungen praktisch nicht beeinflußt, und sie zeigen somit eine befriedigende Nuancenkonstanz.

Die mit den Verbindungen (1) erhaltenen Färbungen oder Drucke auf Cellulosefasermaterialien besitzen beachtliche Echtheiten; hiervon sind insbesondere die wichtigsten Fabrikations- und Gebrauchsechtheiten hervorzuheben, wie die Lichtechtheiten auf feuchtem und trockenem Fasermaterial, die Waschechtheit bei 60°C und 95°C, die Sodakochechtheit, die saure und alkalische Walkechtheit, die Wasserechtheit, die Seewasserechtheit, die saure Überfärbeechtheit, die alkalische und saure Schweißechtheit, die Plissier-, Bügel- und Reibechtheit sowie die Chlorbadewasser- und die Abgasechtheit. Kupfersalze verändern auch in Gegenwart von peroxidhaltigen Waschmitteln die Nuance der Färbungen und Drucke nicht. Ebenso wird die Nuance durch eine Kunstharzausrüstung nicht verändert. Weiterhin zeigen die Verbindungen (1) keine Phototropie.

Sowohl die natürlichen als auch die synthetischen Polyamidfasermaterialien werden mit den Verbindungen (1) bevorzugt aus saurem, wäßrigem Färbebad oder saurer, wäßriger Färbeflotte gefärbt. Vorzugsweise wird der gewünschte pH-Wert des Färbebades oder der Färbeflotte mit Essigsäure oder Essigsäure und Ammoniumacetat oder Natriumacetat eingestellt. Um eine gute Egalität der Färbungen zu erreichen oder deren Egalität zu verbessern, ist es vorteilhaft, übliche Egalisierungshilfsmittel, beispielsweise Verbindungen auf Basis eines Umsetzungsproduktes von einem Fettamin, wie beispielsweise Stearylamin, mit einem Alkylenoxid, wie Ethylenoxid, oder eines Umsetzungsproduktes von Cyanurchlorid mit der etwa 3-fach molaren Menge einer Aminobenzolsulfonsäure und/oder einer Aminonaphthalinsulfonsäure, im Färbebad oder in der Färbeflotte mitzuverwenden. Die Färbungen können üblicherweise bei Temperaturen von 60 bis 80°C, vorzugsweise im Ausziehverfahren, oder auch im Klotz-Kaltverweil-Verfahren ausgeführt werden.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter. Die in diesen Beispielen formelmäßig beschriebenen Verbindungen sind in Form der freien Säuren angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als soclhe oder in Form ihrer Salze, vorzugsweise Alkalimetallsalze, in die Synthese eingesetzt werden.

Die für die erfindungsgemäßen Verbindungen angegebenen Absorptionsmaxima (λₘₐₓ) im sichtbaren Bereich wurden anhand derer Alkalimetallsalze in wäßriger Lösung ermittelt. In den Tabellenbeispielen sind die λₘₐₓ-Werte bei der Farbtonangabe in Klammern gesetzt; die Wellenlängenangabe bezieht sich auf nm.

### Beispiel 1

Zu einer Lösung von 0°C mit einem pH-Wert von 7 von 17,4 Teilen Anilin-3-suffonsäure in 200 Teilen Wasser gibt man 8,6 Teile Natriumfluorid, danach unter Einhaltung dieser Temperatur 15 Vol.-Teile einer konzentrierten wäßrigen Salzsäure und anschließend unter Einhaltung einer Temperatur von 0 bis 5°C innerhalb von etwa 2 Minuten stetig 13,6 Vol.-Teile Trifluortriazin. Man rührt den Ansatz noch etwa 5 Minuten weiter und gibt sodann eine wäßrige Lösung mit einem pH-Wert von 6 von 19,3 Teilen 1,3-Diaminobenzol-4-sulfonsäure in 100 Teilen Wasser hinzu; die Umsetzung erfolgt unter weiterem Rühren unter Einhaltung eines pH-Wertes von 5,5 und einer Temperatur zwischen 5 und 10°C.

Zu der so erhaltenen Lösung der Kupplungskomponente gibt man die auf üblichem Wege erhaltene salzsaure, wäßrige Lösung des Diazoniumsalzes aus 26,6 Teilen 4-(β-Sulfatoethylsulfonyl)-anilin; die Kupplungsreaktion erfolgt bei einem pH-Wert zwischen 3 und 6,5 und einer Temperatur zwischen 10 und 15°C.

Die erfindungsgemäße Azoverbindung wird in üblicher Weise aus der Syntheselösung isoliert, beispielsweise durch Aussalzen mit Natriumchlorid. Nach Filtration und Trocknen erhält man ein gelbes, elektrolythaltiges (natriumchloridhaltiges) Pulver der erfindungsgemäßen Verbindung der Formel (in Form der freien Säure geschrieben)

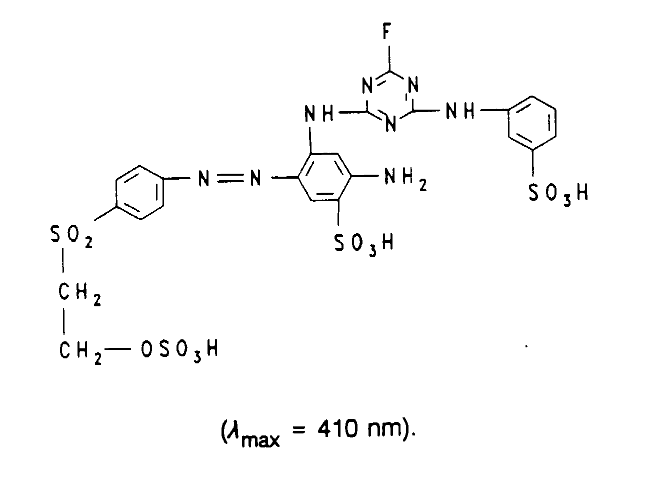

Die erfindungsgemäße Monoazoverbindung besitzt sehr gute faserreaktive Farbstoffeigenschaften und liefert nach den in der Technik für faserreaktive Farbstoffe üblichen Applikations- und Fixierverfahren auf den in der Beschreibung genannten Materialien, insbesondere Cellulosefasermaterialien, farbstarke gelbe Färbungen mit guten Echtheitseigenschaften, von denen insbesondere die Chlor-, Licht-, Schweißlicht- und Waschechtheiten hervorgehoben werden können.

### Beispiele 2 bis 31

In den nachfolgenden Tabellenbeispielen sind weitere erfindungsgemäße Azofarbstoffe entsprechend der allgemeinen Formel (A) 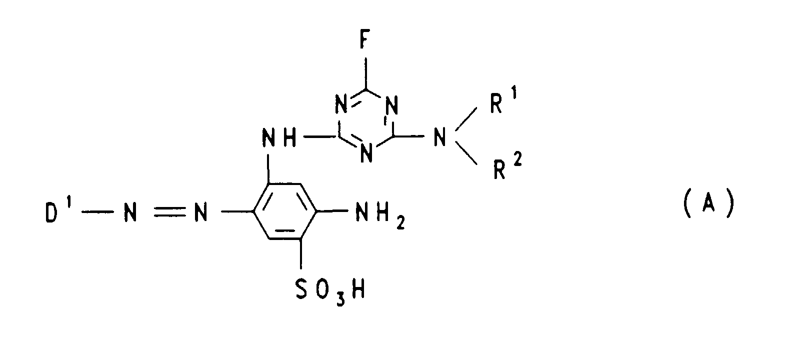 mit Hilfe ihrer Komponenten beschrieben. Sie lassen sich in erfindungsgemäßer Weise, beispielsweise analog dem obigen Ausführungsbeispiel, unter Einsatz der aus dem jeweiligen Tabellenbeispiel in Verbindung mit der allgemeinen Formel (A) ersichtlichen Komponenten (Cyanurfluorid, 1,3-Diamino-benzol-6-sulfonsäure, dem Amin der allgemeinen Formel HNR¹ R² und dem Diazoniumsalz des Amins der allgemeinen Formel D¹-NH₂) herstellen. Sie besitzen sehr gute faserreaktive Farbstoffeigenschaften und färben die in der Beschreibung genannten Materialien, insbesondere Cellulosefasermaterialien, wie Baumwolle, in den in dem jeweiligen Tabellenbeispiel (hier für Baumwolle) angegebenen Farbton in hoher Farbstärke und mit guten Echtheiten.

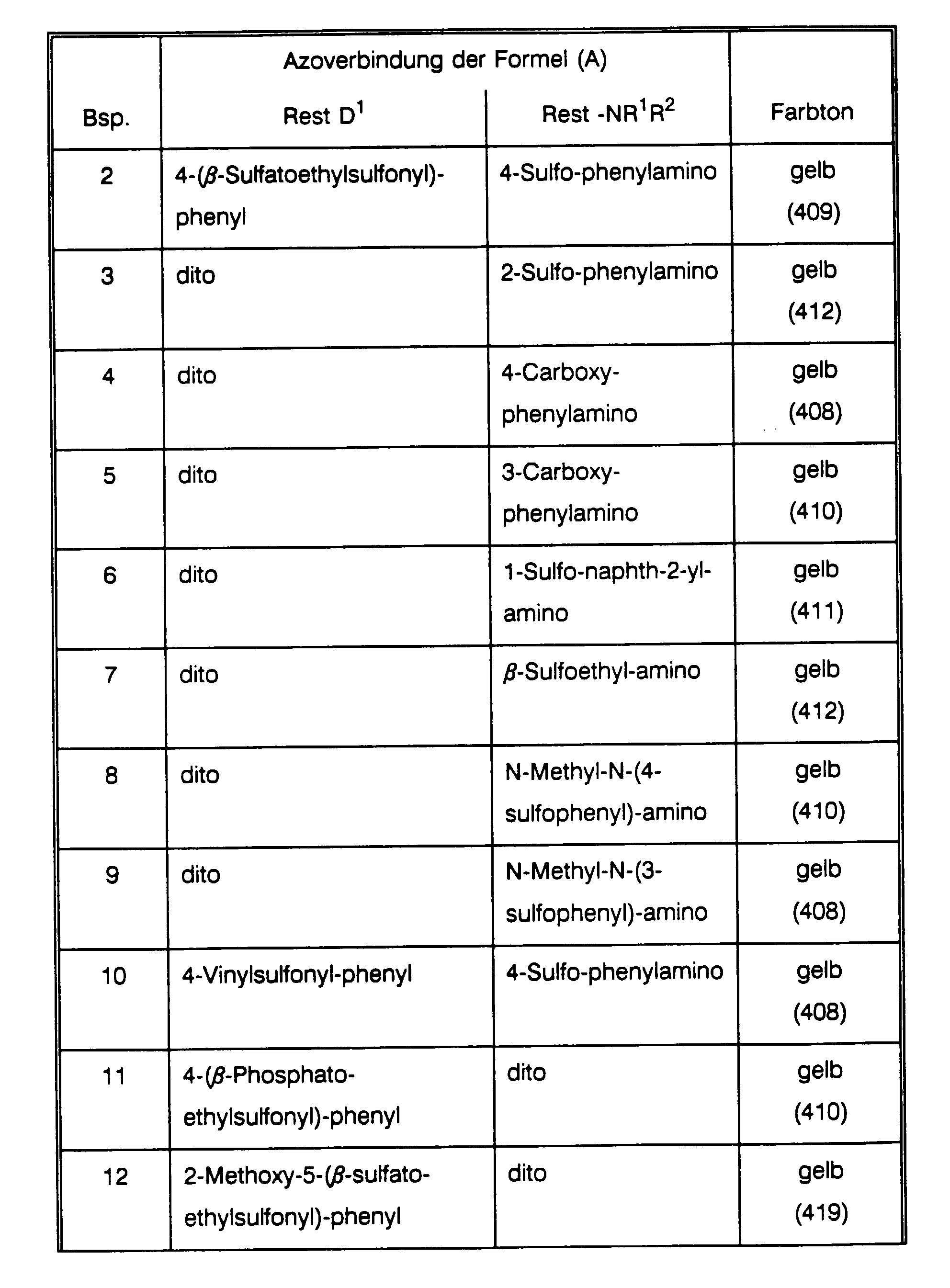

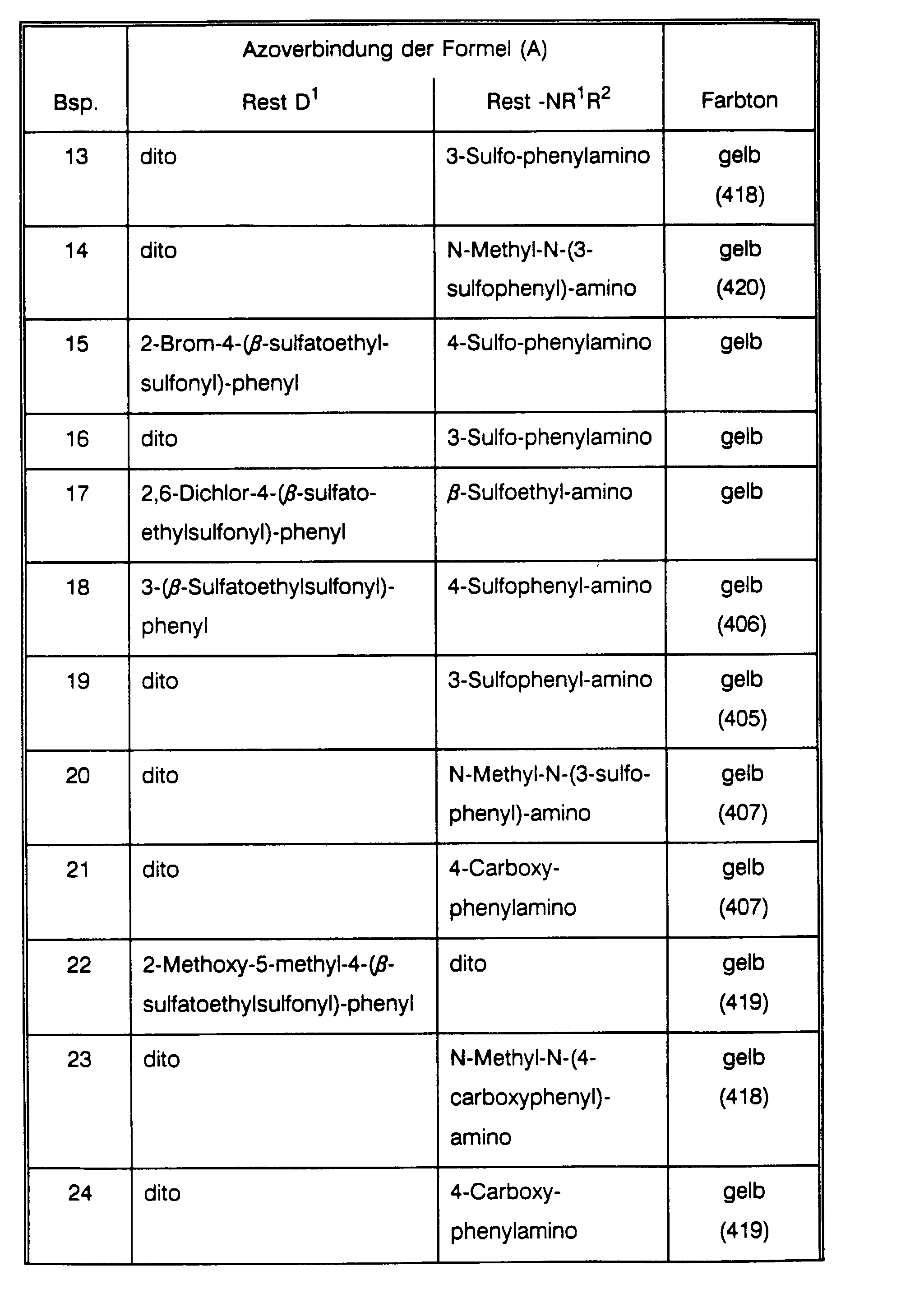

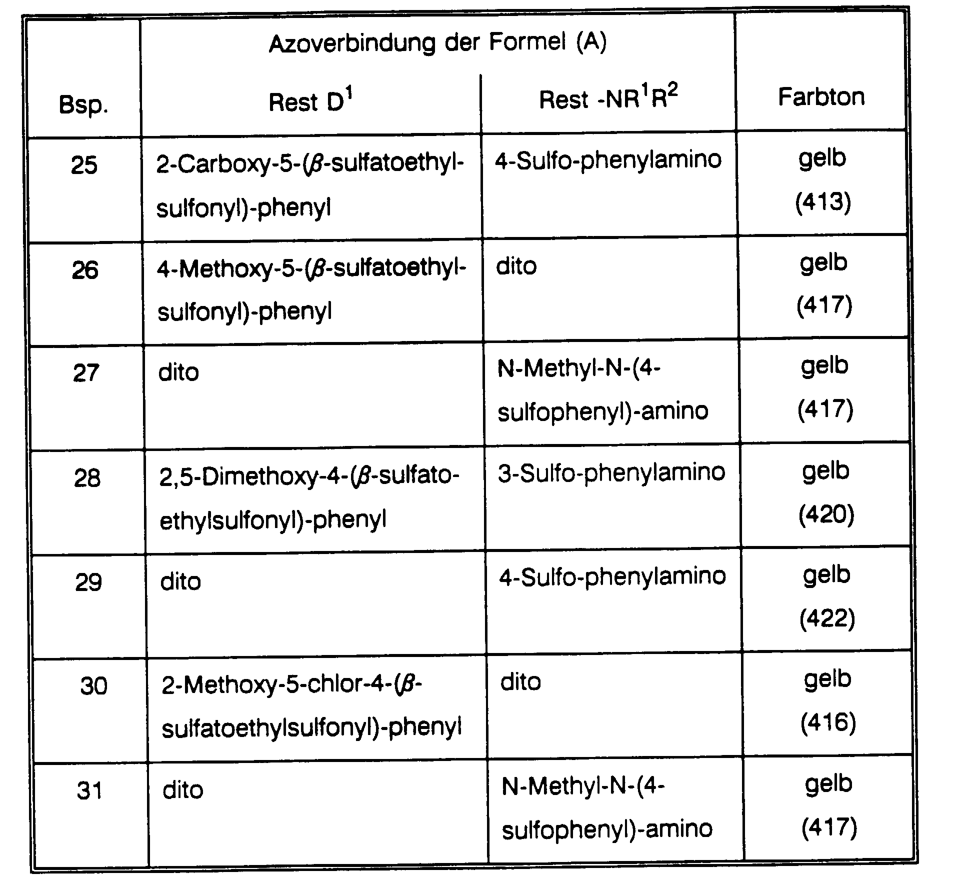

## Patentansprüche

1. Verbindung entsprechend der allgemeinen Formel (1) 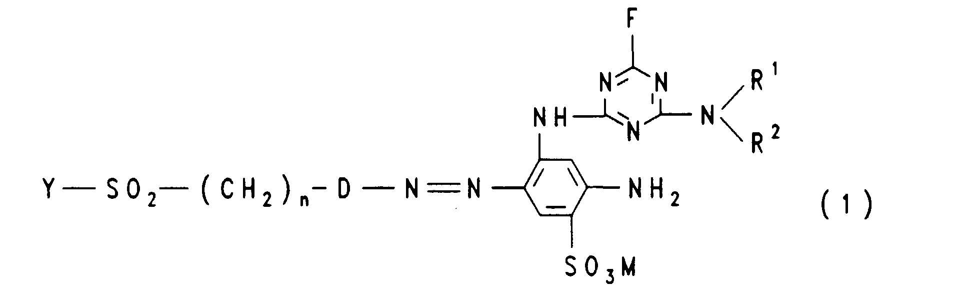 in welcher bedeuten:
Y ist Vinyl oder ist Ethyl, das in β-Stellung einen Substituenten enthält, der durch ein Alkali unter Bildung der Vinylgruppe eliminierbar ist, oder ist β-Hydroxyethyl;
n ist die Zahl Null, 1 oder 2;
D ist Phenylen, das durch 1 oder 2 Substituenten aus der Gruppe Alkyl von 1 bis 4 C-Atomen, Alkoxy von 1 bis 4 C-Atomen, Sulfo, Chlor, Brom, Fluor, Carboxy, Nitro, Hydroxy, Alkanoylamino von 2 bis 5 C-Atomen, Benzoylamino und durch Substituenten aus der Gruppe Methyl, Ethyl, Methoxy, Ethoxy, Chlor und Sulfo substituiertes Benzoylamino oder durch 1 oder 2 dieser Substituenten und eine Gruppe der allgemeinen Formel Y-SO₂-(CH₂)ₘ-, in welcher Y eine der oben genannten Bedeutungen hat und m die Zahl 1 oder 2 ist, oder durch eine Gruppe der Formel Y-SO₂-(CH₂)ₘ- substituiert sein kann, oder
D ist Naphthylen, das durch 1 oder 2 Substituenten aus der Gruppe Alkyl von 1 bis 4 C-Atomen, Alkoxy von 1 bis 4 C-Atomen, Chlor, Brom, Fluor, Carboxy, Sulfo, Nitro, Hydroxy, Alkanoylamino von 2 bis 5 C-Atomen, Benzoylamino und durch Substituenten aus der Gruppe Alkyl von 1 bis 4 C-Atomen, Alkoxy von 1 bis 4 C-Atomen, Chlor und Sulfo substituiertes Benzoylamino substituiert sein kann, oder
D ist Benzthiazol-2-yl, das im carbocyclischen Ring die angegebene Gruppe der Formel Y-SO₂-(CH₂)ₙ- gebunden enthält und in diesem Benzolkern durch einen weiteren Substituenten aus der Gruppe Alkyl von 1 bis 4 C-Atomen, Alkoxy von 1 bis 4 C-Atomen, Sulfo, Nitro, Chlor, Brom, Hydroxy, Alkanoylamino von 2 bis 5 C-Atomen, Benzoylamino und durch Substituenten aus der Gruppe Alkyl von 1 bis 4 C-Atomen, Alkoxy von 1 bis 4 C-Atomen, Chlor und Sulfo substituiertes Benzoylamino substituiert sein kann;
M ist Wasserstoff oder das Moläquivalent eines Metalls, vorzugsweise eines Alkalimetalls oder Erdalkalimetalls;
R¹ ist Wasserstoff oder Alkyl von 1 bis 4 C-Atomen oder ist Phenyl oder ist Alkyl von 1 bis 4 C-Atomen, das durch 1 oder 2 nicht-ionogene und/oder anionische Substituenten substituiert ist;
R² ist Alkyl von 1 bis 4 C-Atomen oder ist Phenyl oder ist Alkyl von 1 bis 4 C-Atomen, das durch 1 oder 2 nicht-ionogene und/oder anionische Substituenten substituiert ist, oder ist Phenyl, das durch 1,2 oder 3 Substituenten aus der Gruppe Methyl, Ethyl, Methoxy, Ethoxy, Chlor, Cyano, Nitro, Sulfo, Carboxy und Carbamoyl, bevorzugt durch 1 oder 2 Substituenten aus der Gruppe Sulfo und Carboxy, substituiert ist, oder ist Naphthyl, das durch 1,2 oder 3 Sulfogruppen oder durch 1 oder 2 Carboxygruppen oder durch 1 oder 2 Sulfogruppen und eine Carboxygruppe substituiert sein kann.

2. Verbindung nach Anspruch 1, dadurch gekennzeichnet, daß D Phenylen ist, das unsubstituiert oder durch 1 oder 2 Substituenten aus der Gruppe Alkyl von 1 bis 4 C-Atomen, Alkoxy von 1 bis 4 C-Atomen, Chlor und Brom substituiert ist.

3. Verbindung nach Anspruch 1, dadurch gekennzeichnet, daß D Naphthylen ist, das unsubstituiert oder durch eine oder zwei Sulfogruppen substituiert ist.

4. Verbindung nach mindestens einem der Ansprüche 1 bis 3, dadurch gekennzeichnet, daß R¹ Wasserstoff und R² Alkyl von 1 bis 4 C-Atomen oder Alkyl von 2 bis 4 C-Atomen, das durch Carboxy oder Sulfo substituiert ist, oder Phenyl ist, das durch Substituenten aus der Gruppe Carboxy und Sulfo substituiert sein kann.

5. Verbindung nach mindestens einem der Ansprüche 1 bis 3, dadurch gekennzeichnet, daß R¹ Wasserstoff und R² Alkyl von 2 bis 4 C-Atomen ist, das durch Sulfo substituiert ist, oder sulfosubstituiertes Phenyl ist.

6. Verbindung nach mindestens einem der Ansprüche 1 bis 5, dadurch gekennzeichnet, daß n die Zahl Null bedeutet.

7. Verbindung nach Anspruch 1 entsprechend der allgemeinen Formel (1) 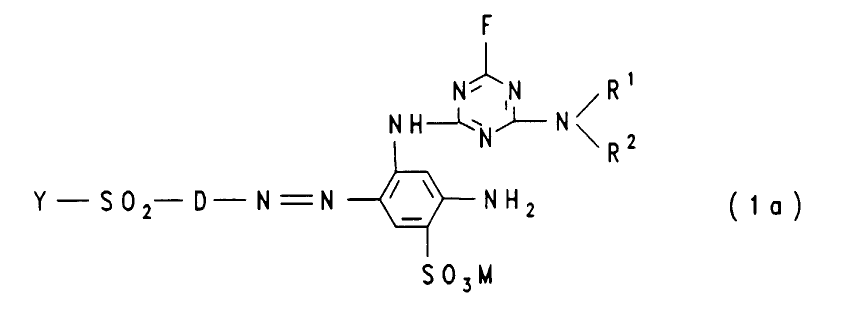 in welcher R¹, R², M und Y eine der in Anspruch 1 genannten Bedeutungen besitzt und D Phenylen oder durch 1 oder 2 Substituenten aus der Gruppe Methyl, Ethyl, Methoxy, Ethoxy, Chlor und Brom substituiertes Phenylen oder Naphthylen ist, das durch eine Sulfogruppe substituiert sein kann.

8. Verbindung nach Anspruch 7, dadurch gekennzeichnet, daß D Phenylen ist, das durch 1 oder 2 Substituenten aus der Gruppe Methyl, Methoxy, und Ethoxy substituiert sein kann.

9. Verbindung nach Anspruch 7, dadurch gekennzeichnet, daß D Phenylen ist.

10. Verbindung nach mindestens einem der Ansprüche 7 bis 9, dadurch gekennzeichnet, daß R¹ Wasserstoff und R² Monosulfo-phenyl oder Disulfo-phenyl ist.

11. Verfahren zur Herstellung einer Verbindung der allgemeinen Formel (1) gemäß Anspruch 1, dadurch gekennzeichnet, daß man die Diazoniumverbindung einer Aminoverbindung der allgemeinen Formel (2)
Y - SO₂ - (CH₂)ₙ - D - NH₂ (2)
in welcher D, Y und n eine der in Anspruch 1 genannten Bedeutungen besitzt, mit einer Verbindung der allgemeinen Formel (3) 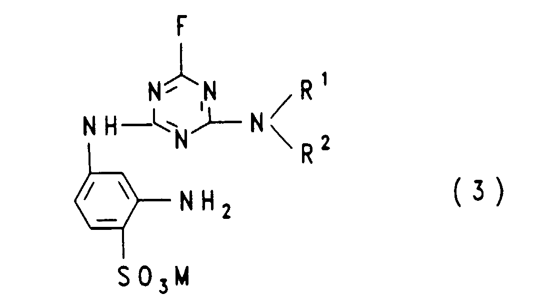 in welcher R¹, R² und M eine der in Anspruch 1 genannten Bedeutungen besitzt, kuppelt und gewünschtenfalls im Falle, daß Y β-Hydroxyethyl ist, die Gruppe Y-SO₂- in üblicher Weise mit Hilfe eines Sulfatierungsmittels in die Azoverbindung der allgemeinen Formel (1) mit Y gleich β-Sulfatoethyl oder mittels eines Phosphatierungsmittels in die Azoverbindung der allgemeinen Formel (1) mit Y gleich β-Phosphatoethyl überführt.

12. Verwendung einer Verbindung von Anspruch 1 zum Färben (einschließlich Bedrucken) von hydroxy- und/oder carbonamidgruppenhaltigem Material, insbesondere Fasermaterial.

13. Verfahren zum Färben (einschließlich Bedrucken) von hydroxy- und/oder carbonamidgruppenhaltigem Material, vorzugsweise Fasermaterial, bei welchem man einen Farbstoff auf das Material aufbringt oder in das Material einbringt und den Farbstoff mittels Wärme oder mit Hilfe eines alkalisch wirkenden Mittels oder durch beide Maßnahmen auf bzw. in dem Material fixiert, dadurch gekennzeichnet, daß man als Farbstoff eine Verbindung von Anspruch 1 verwendet.

## Claims

1. A compound of the formula (1) 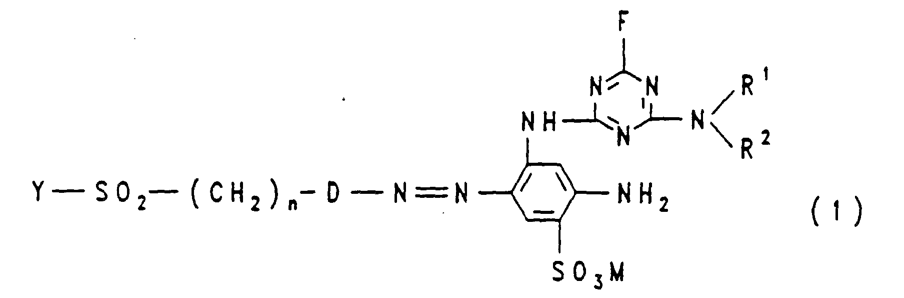 in which
Y is vinyl or is ethyl which contains a substituent in the β position which can be eliminated by an alkali with the formation of a vinyl group, or is β-hydroxyethyl;
n is the number zero, 1 or 2;
D is phenylene which can be substituted by 1 or 2 substituents from the group consisting of alkyl of 1 to 4 carbon atoms, alkoxy of 1 to 4 carbon atoms, sulfo, chlorine, bromine, fluorine, carboxyl, nitro, hydroxyl, alkanoylamino of 2 to 5 carbon atoms, benzoylamino and benzoylamino which is substituted by substituents from the group consisting of methyl, ethyl, methoxy, ethoxy, chlorine and sulfo, or by 1 or 2 of these substituents and a group of the formula Y-SO₂-(CH₂)ₘ- in which Y has one of the abovementioned meanings and m is the number 1 or 2, or by a group of the formula Y-SO₂-(CH₂)ₘ-, or
D is naphthylene which can be substituted by 1 or 2 substituents from the group consisting of alkyl of 1 to 4 carbon atoms, alkoxy of 1 to 4 carbon atoms, chlorine, bromine, fluorine, carboxyl, sulfo, nitro, hydroxyl, alkanoylamino of 2 to 5 carbon atoms, benzoylamino and benzoylamino which is substituted by substituents from the group consisting of alkyl of 1 to 4 carbon atoms, alkoxy of 1 to 4 carbon atoms, chlorine and sulfo, or
D is benzothiazol-2-yl which contains the group mentioned of the formula Y-SO₂-(CH₂)ₙ- bound to the carbocyclic ring and can be substituted in this benzene ring by a further substituent from the group consisting of alkyl of 1 to 4 carbon atoms, alkoxy of 1 to 4 carbon atoms, sulfo, nitro, chlorine, bromine, hydroxyl, alkanoylamino of 2 to 5 carbon atoms, benzoylamino and benzoylamino which is substituted by substituents from the group consisting of alkyl of 1 to 4 carbon atoms, alkoxy of 1 to 4 carbon atoms, chlorine and sulfo;
M is hydrogen or the mole equivalent of a metal, preferably of an alkali metal or alkaline earth metal;
R¹ is hydrogen or alkyl of 1 to 4 carbon atoms, or is phenyl or is alkyl of 1 to 4 carbon atoms, which is substituted by 1 or 2 nonionic and/or anionic substituents;
R² is alkyl of 1 to 4 carbon atoms, or is phenyl or is alkyl of 1 to 4 carbon atoms, which is substituted by 1 or 2 nonionic and/or anionic substituents, or is phenyl which is substituted by 1, 2 or 3 substituents from the group consisting of methyl, ethyl, methoxy, ethoxy, chlorine, cyano, nitro, sulfo, carboxyl and carbamoyl, preferably by 1 or 2 substituents from the group consisting of sulfo and carboxyl, or is naphthyl which can be substituted by 1, 2 or 3 sulfo groups or by 1 or 2 carboxyl groups or by 1 or 2 sulfo groups and a carboxyl group.

2. A compound as claimed in claim 1, wherein D is phenylene which is unsubstituted or substituted by 1 or 2 substituents from the group consisting of alkyl of 1 to 4 carbon atoms, alkoxy of 1 to 4 carbon atoms, chlorine and bromine.

3. A compound as claimed in claim 1, wherein D is naphthylene which is unsubstituted or substituted by one or two sulfo groups.

4. A compound as claimed in at least one of claims 1 to 3, wherein R¹ is hydrogen and R² is alkyl of 1 to 4 carbon atoms or alkyl of 2 to 4 carbon atoms which is substituted by carboxyl or sulfo, or is phenyl which can be substituted by substituents from the group consisting of carboxyl and sulfo.

5. A compound as claimed in at least one of claims 1 to 3, wherein R¹ is hydrogen and R² is alkyl of 2 to 4 carbon atoms which is substituted by sulfo, or is sulfo-substituted phenyl.

6. A compound as claimed in at least one of claims 1 to 5, wherein n is the number zero.

7. A compound as claimed in claim 1 of the formula (1) 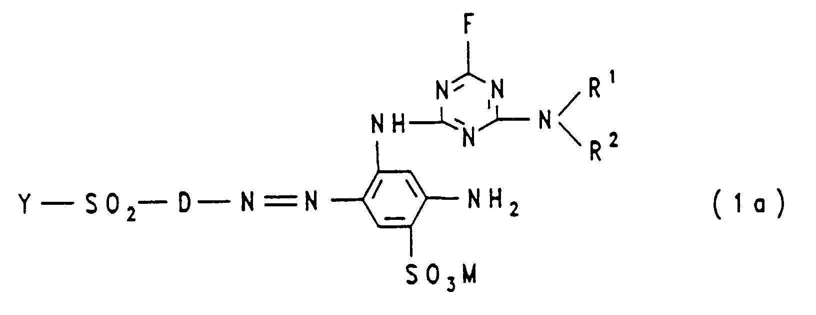 in which R¹, R², M and Y have one of the meanings mentioned in claim 1 and D is phenylene or phenylene which is substituted by 1 or 2 substituents from the group consisting of methyl, ethyl, methoxy, ethoxy, chlorine and bromine, or is naphthylene which can be substituted by a sulfo group.

8. A compound as claimed in claim 7, wherein D is phenylene which can be substituted by 1 or 2 substituents from the group consisting of methyl, methoxy and ethoxy.

9. A compound as claimed in claim 7, wherein D is phenylene.

10. A compound as claimed in at least one of claims 7 to 9, wherein R¹ is hydrogen and R² is monosulfophenyl or disulfophenyl.

11. A process for the preparation of a compound of the formula (1) as claimed in claim 1, which comprises coupling the diazonium compound of an amino compound of the formula (2)
Y-SO₂- (CH₂)ₙ-D-NH₂ (2)
in which D, Y and n have one of the meanings mentioned in claim 1, to a compound of the formula (3) 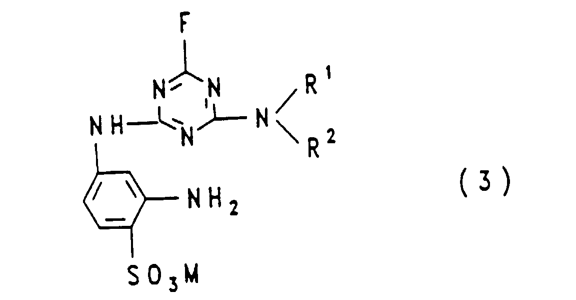 in which R¹, R² and M have one of the meanings mentioned in claim 1, and, if desired, converting the group Y-SO₂-, in the case where Y is β-hydroxyethyl, in the usual manner into the azo compound of the formula (1) where Y is β-sulfatoethyl by means of a sulfating agent or into the azo compound of the formula (1) where Y is β-phosphatoethyl by means of a phosphating agent.

12. The use of a compound of claim 1 for the dyeing (including printing) of hydroxyl- and/or carboxamido-containing material, in particular fiber material.

13. A process for the dyeing (including printing) of hydroxyl- and/or carboxamido-containing material, preferably fiber material, in which a dye is applied to the material or incorporated in the material and the dye is fixed on or in the material by means of heat or by means of an alkaline agent or by both measures, which comprises using a compound of claim 1 as the dye.

## Revendications

1. Composé répondant à la formule générale (1) 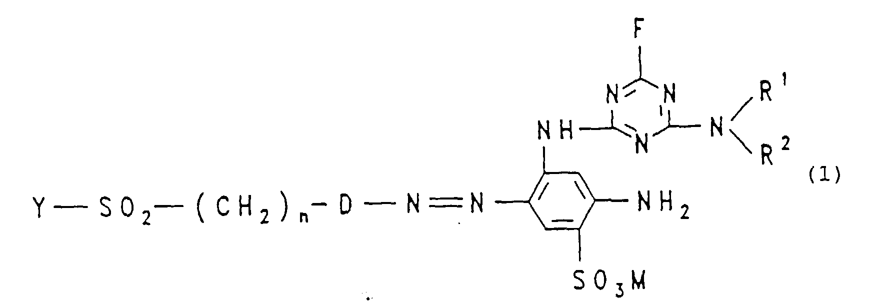 dans laquelle représentent :
Y est vinyle ou éthyle qui contient en position β un substituant éliminable par un alcali en formant le groupe vinyle, ou est β-hydroxyéthyle ;
n vaut 0, 1 ou 2 ;
D est phénylène qui peut être substitué par un ou deux substituants pris dans le groupe comportant alkyle avec 1 à 4 atomes de carbone, alcoxy avec 1 à 4 atomes de carbone, sulfo, chloro, bromo, fluoro, carboxy, nitro, hydroxy, alcanoylamino avec 2 à 5 atomes de carbone, benzoylamino ou benzoylamino substitué par des substituants pris dans le groupe comportant méthyle, éthyle, méthoxy, éthoxy, chloro et sulfo, ou par un ou deux de ces substituants et un groupe de formule générale
Y-SO₂-(CH₂)ₘ-, dans laquelle Y a l'une des significations citées ci-dessus et m est le nombre 1 ou 2, ou par un groupe de formule Y-SO₂-(CH₂)ₘ-, ou
D est naphtylène qui peut être substitué par 1 ou 2 substituants pris dans le groupe comportant alkyle avec 1 à 4 atomes de carbone, alcoxy avec 1 à 4 atomes de carbone, chloro, bromo, fluoro, carboxy, sulfo, nitro, hydroxy, alcanoylamino avec 2 à 5 atomes de carbone, benzoylamino ou benzoylamino substitué par des substituants pris dans le groupe comportant alkyle avec 1 à 4 atomes de carbone, alcoxy avec 1 à 4 atomes de carbone, chloro et sulfo, ou
D est benzothiazol-2-yle qui contient lié au cycle carbocyclique le groupe indiqué de formule Y-SO₂-(CH₂)ₙ- et peut être substitué dans ce cycle de benzène par un autre substituant pris dans le groupe comportant alkyle avec 1 à 4 atomes de carbone, alcoxy avec 1 à 4 atomes de carbone, sulfo, nitro, chloro, bromo, hydroxy, alcanoylamino avec 2 à 5 atomes de carbone, benzoylamino et benzoylamino substitué par des substituants pris dans le groupe comportant alkyle avec 1 à 4 atomes de carbone, alcoxy avec 1 à 4 atomes de carbone, chloro et sulfo ;
M est l'hydrogène ou l'équivalent en moles d'un métal, de préférence d'un métal alcalin ou alcalino-terreux ;
R¹ est l'hydrogène ou alkyle avec 1 à 4 atomes de carbone, ou est phényle ou alkyle avec 1 à 4 atomes de carbone, qui est substitué par 1 ou 2 substituants non ionogènes et/ou anioniques ;
R² est alkyle avec 1 à 4 atomes de carbone, ou est phényle ou alkyle avec 1 à 4 atomes de carbone, qui est substitué par 1 ou 2 substituants non-ionogènes et/ou anioniques, ou est phényle, qui est substitué par 1, 2 ou 3 substituants pris dans le groupe comportant méthyle, éthyle, méthoxy, éthoxy, chloro, cyano, nitro, sulfo, carboxy et carbamoyle, de préférence par 1 ou 2 substituants pris dans le groupe comportant sulfo et carboxy, ou est naphtyle qui peut être substitué par 1, 2 ou 3 groupes sulfo ou 1 ou 2 groupes carboxy ou par un 1 ou 2 groupes sulfo et un groupe carboxy.

2. Composé selon la revendication 1, caractérisé en ce que D est phénylène, qui est non substitué ou substitué par 1 ou 2 substituants pris dans le groupe comportant alkyle avec 1 à 4 atomes de carbone, alcoxy avec 1 à 4 atomes de carbone, le chlore et le brome.

3. Composé selon la revendication 1, caractérisé en ce que D est naphtylène, qui est non substitué ou substitué par 1 ou 2 groupes sulfo.

4. Composé selon au moins l'une des revendications 1 à 3, caractérisé en ce que R¹ est l'hydrogène et R² est alkyle avec 1 à 4 atomes de carbone ou alkyle avec 2 à 4 atomes de carbone qui est substitué par carboxy ou sulfo, ou est phényle, qui est substitué par des substituants pris dans le groupe comportant carboxy et sulfo.

5. Composé selon au moins l'une des revendications 1 à 3, caractérisé en ce que R¹ est l'hydrogène et R² est alkyle avec 2 à 4 atomes de carbone, qui est substitué par sulfo, ou phényle substitué par sulfo.

6. Composé selon au moins l'une des revendications 1 à 5, caractérisé en ce n vaut 0.

7. Composé selon la revendication 1 répondant à la formule générale (1) 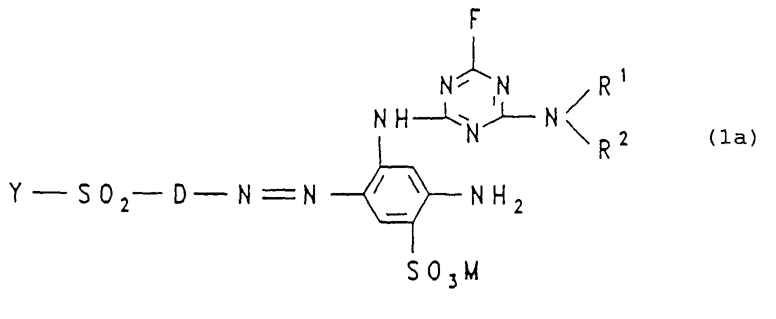 dans laquelle R¹, R², M et Y ont les significations données dans la revendication 1, et D représente phénylène ou phénylène substitué par 1 ou 2 substituants pris dans le groupe comportant méthyle, éthyle, méthoxy, éthoxy, chloro et bromo, ou est naphtylène qui peut être substitué par un groupe sulfo.

8. Composé selon la revendication 7, caractérisé en ce que D est phénylène, qui est substitué par 1 ou 2 substituants pris dans le groupe méthyle, méthoxy et éthoxy.

9. Composé selon la revendication 7, caractérisé en ce que D est phénylène.

10. Composé selon au moins l'une des revendications 7 à 9, caractérisé en ce que R¹ est l'hydrogène et R² est monosulfophényle ou disulfophényle.

11. Procédé pour la préparation d'un composé de formule générale (1) selon la revendication 1, caractérisé en ce que l'on copule le composé de diazonium d'un composé amino de formule générale (2) :
Y-SO₂-(CH₂)ₙ-D-NH₂
dans laquelle D, Y et n ont les significations données dans la revendication 1, avec un composé de formule générale (3) : 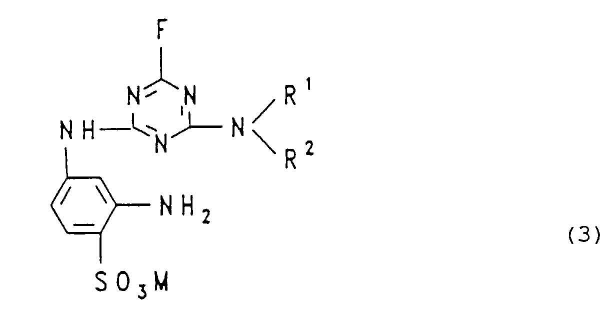 dans laquelle R¹, R² et M ont l'une des significations données dans la revendication 1 et, si on le désire, dans le cas où Y est β-hydroxyéthyle, on transforme le groupe Y-SO₂- de façon usuelle à l'aide d'un agent de sulfatation en le composé azoïque de formule générale (1) avec Y étant β-sulfato-éthyle ou à l'aide d'un agent de phosphatation en le composé azoïque de formule générale (1) avec Y étant β-phosphatoéthyle.

12. Utilisation d'un composé de la revendication 1 pour la teinture (y compris l'impression) de matière contenant des groupes hydroxy et/ou carboxamido, plus particulièrement de matières fibreuses.

13. Procédé pour la teinture (y compris l'impression) de matières contenant des groupes hydroxy et/ou carboxamido, de préférence de matières fibreuses, selon lequel on applique sur la matière ou on incorpore dans la matière un colorant et on fixe la matière colorante à l'aide de la chaleur ou à l'aide d'un agent alcalin ou à l'aide de ces deux mesures sur, respectivement dans, la matière, caractérisé en ce que la matière colorante est un composé de la revendication 1.
